# EUROPEAN PATENT APPLICATION

(11) **EP 0 611 769 A1**
(43) Date of publication of application: **24.08.1994**
(21) Application number: 93400385.6
(22) Date of filing: 16.02.1993
(51) Int. Cl.: C07F 7/08, A61K 31/695

(54) **Silylated acetylcholinesterase inhibitors**

(71) Applicant: MERRELL DOW PHARMACEUTICALS INC., Cincinnati Ohio 45215-6300 (US)
(72) Inventor: Collard, Jean-Noel, F-67310 Balbronn (FR); Hornsperger, Jean-Marie, F-67210 Griesheim-Pres-Molsheim (FR)
(74) Representative: Gillard, Marie-Louise

(57) **Abstract**

Fluorinated silylated aromatic compounds possessing anticholinesterase activity and methods of treating Degenerative Dementias using these compounds.

## Description

This invention relates to fluorinated silylated aromatic compounds, their intermediates, methods of use in treating diseases associated with deficiencies of cholinergic transmission in the central nervous system and methods for their preparation.

Compounds of the present invention have the following Formula I: 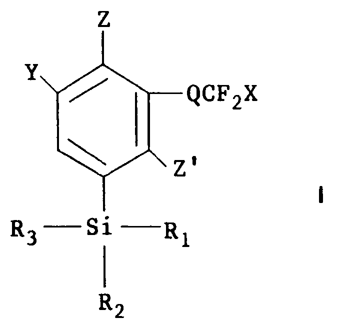
stereoisomers or mixtures thereof, and pharmaceutically acceptable salts thereof, wherein:
each of Z and Z' are independently H or F, provided that at least one of Z or Z' is F;
Q is 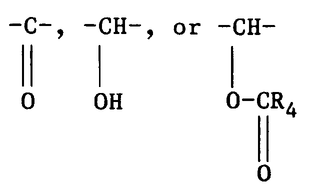
X is H, Br, Cl, F or CF₃;
Y is H, OH, (C₁₋₆) alkyl, -(CH₂)ₘOR₅_{,} hydroxy (C₁₋₆) alkyl, (CH₂)ₙNR₆R₆', azido, CN, CO₂R₄, COR₆, SO₃H, Br, Cl, F, NO₂ or -(CH₂)ₙ SiR₁'R₂'R₃', provided that when both Z and Z' are F then Y is H or F;
R₁, R₂, R₃, R₁', R₂'and R₃' are each independently C₁₋₁₀ alkyl or (CH₂)ₙ aryl;
R₄ is H, (C₁₋₁₀) alkyl, phenyl, benzyl or phenethyl;
R₅ is H, (C₁₋₁₀) alkyl, benzyl or phenethyl;
R₆ and R₆' are independently hydrogen or C₁₋₁₀ alkyl;
m is an integer of 0, 1, 2, 3 or 4; and
n is an integer of 0, 1 or 2.

The compounds of the present invention are used to treat patients having conditions responsive to the acetylcholin-esterase-inhibiting properties of the present compounds such as in the treatment of Degenerative Dementias.

The terms "(C₁₋₆) alkyl" and " (C₁₋₁₀) alkyl" mean straight or branched chain alkyl radicals containing respectively from 1 to 6 carbon atoms and from 1 to 10 carbon atoms, including, but not limited to, methyl, ethyl, n-propyl, iso-propyl, n-butyl, iso-butyl, sec-butyl, t-butyl, n-pentyl, 1-methylbutyl, 2,2-dimethylbutyl, 2-methylpentyl, 2,2-dimethylpropyl, n-hexyl and so on. Likewise, the terms "(CH₂)ₙ" or "(CH₂)ₘ" may represent alkylene chains which may be branched or straight-chained.

"Hydroxy(C₁₋₆) alkyl" means a (C₁₋₆) alkyl group having from 1 to 3 hydroxy substituents thereon. Preferably, there is only one hydroxy substituent at the alpha position (attached to the carbon atom which is directly attached to the phenyl).

"Ts" or "tosyl" means 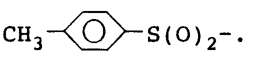
Tosyl derivatives mean 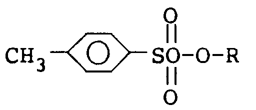
wherein R is C₁₋₆ alkylene.

"Aryl" includes both carbocyclic and heterocyclic moieties of which phenyl, pyridyl, indolyl, indazolyl, furyl and thienyl are of primary interest; these moieties being inclusive of their position isomers such as, for example, 2-, 3-, or 4-pyridyl, 2- or 3-furyl and thienyl, 1-, 2-, or 3-indolyl or the 1- and 3-indazolyl, as well as the dihydro and tetrahydro analogs of the furyl and thienyl moieties. Also included within the term "aryl" are such fused carbocyclic moieties as pentalenyl, indenyl, naphthalenyl, azulenyl, heptalenyl, acenaphthylenyl, fluorenyl, phenalenyl, phenanthrenyl, anthracenyl, acephenanthrylenyl, aceanthrylenyl, triphenylenyl, pyrenyl, chrysenyl and naphthacenyl. Also included within the term "aryl" are such other heterocyclic radicals as 2- or 3-benzo[b]thienyl, 2- or 3-naphtho-[2,3-b]thienyl, 2- or 3-thianthrenyl, 2H-pyran-3-(or 4- or 5-)yl, 1-isobensofuranyl, 2H-chromenyl-3-yl, 2-or 3-phenoxathiinyl, 2- or 3-pyrrolyl, 4- or 3-pyrazolyl, 2-pyrazinyl, 2-pyrimidinyl, 3-pyridazinyl, 2-indolizinyl, 1-isoindolyl, 4H-quinolizin-2-yl, 3-isoquinolyl, 2-quinolyl, 1-phthalazinyl, 1,8-naphthyridinyl, 2-quinoxalinyl, 2-quinazolinyl, 3-cinnolinyl, 2-pteridinyl, 4aH-carbazol-2-yl, 2-carbazolyl, β-carbolin-3-yl, 3-phenanthridinyl, 2-acridinyl, 2-perimidinyl, 1-phenazinyl, 3-isothiazolyl, 2-phenothiazinyl, 3-isoxasolyl, 2-phenoxasinyl, 3-isochromanyl, 7-chromanyl, 2-pyrrolin-3-yl, 2-imidazolidinyl, 2-imidazolin-4-yl, 2-pyrazolidinyl, 3-pyrazolin-3-yl, 2-piperidyl, 2-piperazinyl, 1-indolinyl, 1-isoindolinyl, 3-morpholinyl, benzo[h]isoquinolinyl, and benzo[b]furanyl, including the position isomers thereof except that the heterocyclic moieties cannot be attached directly through their nitrogen atoms. Aryl groups can be substituted or unsubstituted with one, two or three substituents independently selected from C₁₋₆ alkyl, haloalkyl, alkoxy, thioalkoxy, aminoalkylamino, dialkylamino, hydroxy, halo, mercapto, nitro, carboxaldehyde, carboxy, carboalkoxy and carboxamide.

R₁, R₂, R₃, R₁', R₂', and R₃' are each independently selected from C₁₋₁₀ alkyl or (CH₂)ₙ aryl which means that, for example, R₁ could be benzyl while R₂ is methyl. In other words, none of R₁, R₂, R₃, R₁', R₂', or R₃' have to be the same moiety, although this may be the case.

"Stereoisomers" for the compounds of Formula I is a general term for all isomers of individual molecules that differ only in the orientation of their atoms in space. It includes mirror image isomers (enantiomers), geometric isomers (cis/trans), and isomers of compounds with more than one chiral center that are not mirror images of one another (diastereoisomers), whichever forms are applicable to the compound.

The pharmaceutically acceptable salts of the compounds of Formula I include salts formed with non-toxic organic or inorganic acids such as, for example, from the following acids: hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic and benzenesulfonic.

The term "patient" refers to a warm-blooded animal, such as for example rats, mice, dogs, cats, guinea pigs, primates and humans. "Treating" a patient means to prevent or alleviate the patient's disease or condition.

The term "Degenerative Dementia" as used herein means senile dementia, presenile dementia, degenerative dementia of the Alzheimer's type (which includes Alzheimer's Disease) and other types of progressively deteriorating organic mental syndromes in which there is impairment in short-term and long-term memory. The Degenerative Dementia can be mild (impairment of work or social activities but able to live alone), moderate (some degree of supervision needed), or severe (continual supervision required).

Impairment in short-term memory is the inability to learn new information and may be demonstrated by, for example, the patient's inability to remember three objects after five minutes. Long-term memory impairment is the inability to remember information that was known in the past and may be indicated by, for example, the patients' inability to remember past personal information such as their birthplace, address, occupation, what happened yesterday, etc., or the inability to remember facts of common knowledge. There is typically impairment in abstract thinking, impairment in judgment, personality changes or other disturbances of higher cortical functions.

The preparation of the compounds of Formula I may be accomplished in a variety of methods depending upon the specific combinations of variable substituents. The following schemes illustrate only one way these compounds may be made. Other analogous chemical reactions and procedures may be utilized which may be known to those skilled in the art.

### SCHEME A:

To make the sub-generic Formula II: 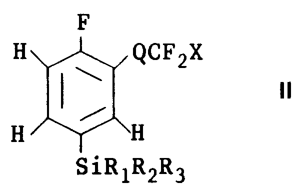
wherein
X=X'or CF₃;
X'=H, Br, Cl, or F; and R₁, R₂, R₃, and
Q are as previously defined. 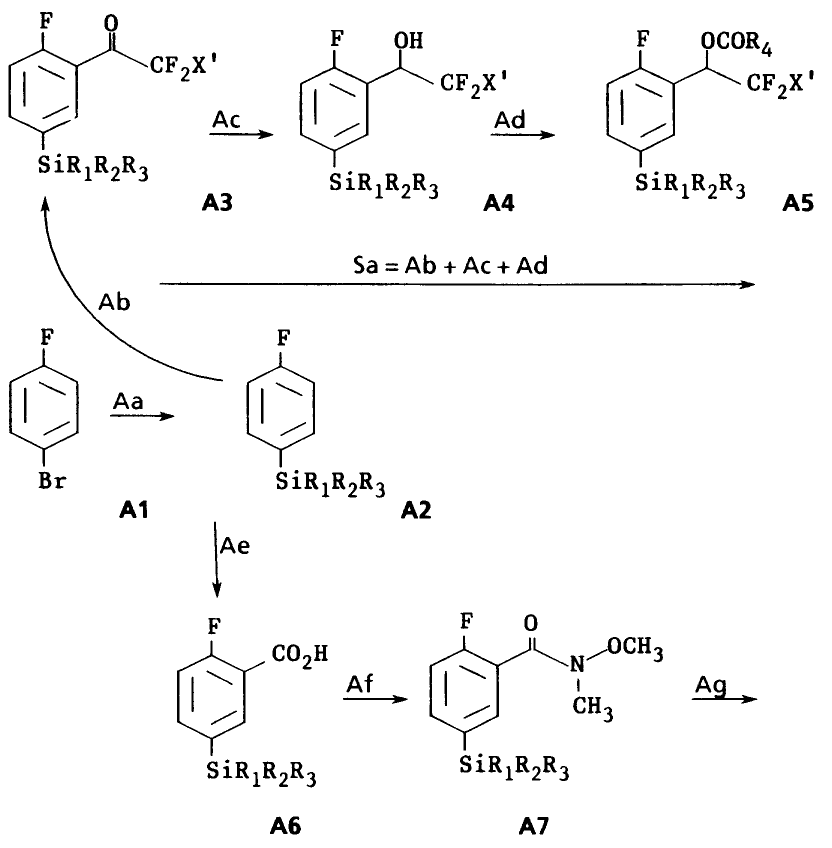 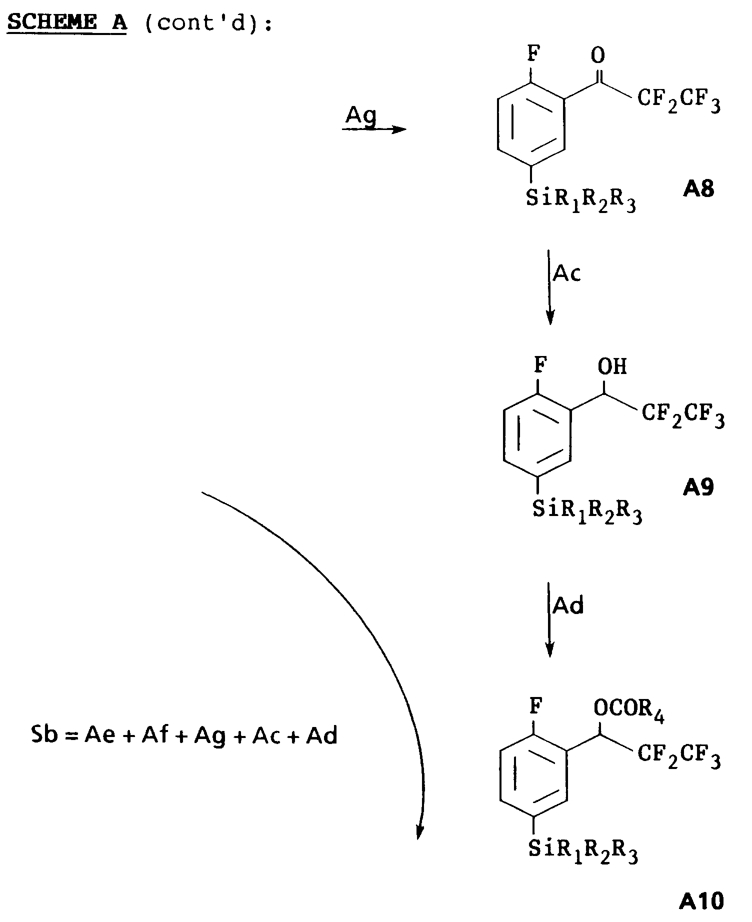
All substituents are as previously defined unless otherwise stated. All starting materials are either commercially available or can be readily prepared by those skilled in the art.

### Reactions:

### Aa: A₁→A₂

The reaction involves the treatment of 4-bromo-1-fluoro benzene with ClSiR₁R₂R₃ in the presence of one equivalent of magnesium in a suitable solvent such as diethylether or tetrahydrofuran at the reflux temperature of the mixture. The silyl derivatives ClSiR₁R₂R₃ are obtained from tetrachlorosilane (SiCl₄) and successive alkylations with organo-magnesium halide derivatives of the appropriate R₁, R₂ and R₃ substituents. For example, SiCl₄ is reacted with R₁Mg halides to produce R₁SiCl₃ compounds which are reacted with R₂Mg halides to produce R₁R₂SiCl₂ compounds which are reacted with R₃Mg halides to produce R₁R₂R₃SiCl compounds.

### Ab: A₂→A₃

Conversion of the 4-fluorotrialkylsilylphenyl compounds to their lithium salts by reaction with an alkyllithium reagent at -50°C in tetrahydrofuran and those intermediates to the desired products by reaction with two equivalents of the appropriate ester (XCF₂CO₂R, with R being preferably ethyl or methyl) or acid lithium salt (XCF₂CO₂Li) followed by hydrolysis with aqueous ammonium chloride.

### Ac: A₃→A₄

Reaction of the ketones preferably using sodium tetraborohydride or sodium cyanoborohydride in ethanol, followed by hydrolysis with aqueous ammonium chloride to produce alcohols.

### Ad: A₄→A₅

Esterification of the alcohols with an acyl chloride [ClCOR₄)] in the presence of triethylamine in a solvent such as dichloromethane to produce esters.

### Ae: A₂→A₆

Following Step Ab the lithium salt intermediates are reacted with carbon dioxide to produce carboxylic acids.

### Af: A₆→A₇

Acids are reacted with isobutylchloroformate in the presence of triethylamine or N-methyl morpholine in tetrahydrofuran to form mixed anhydrides. Addition of molar equivalent amount of N,O-dimethylhydroxylamine to the anhydrides gives dimethylhydroxamic acid derivatives.

### Ag: A₇→A₈

Dimethyl hydroxamic acid derivatives are converted to the pentafluoroketones by treatment with a pentafluoroethyl anion generated *in situ* by contacting pentafluoroethyl iodide with a methyllithium-lithium bromide complex in an inert solvent, preferably diethyl ether. Hydrolysis is performed with aqueous ammonium chloride.

### SCHEME B: to make the subgeneric Formula III:

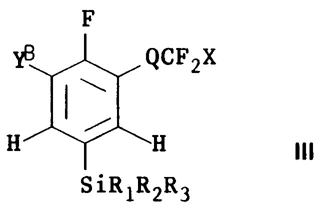

wherein
- X =: X' or CF₃;
- X' =: H, Br, Cl, or F;
- Y^{B} =: OH, NH₂, NR₆R'₆, NO₂, N₃, CN, SO₃H, CO₂R₄, Br, Cl, F, or OR₅.

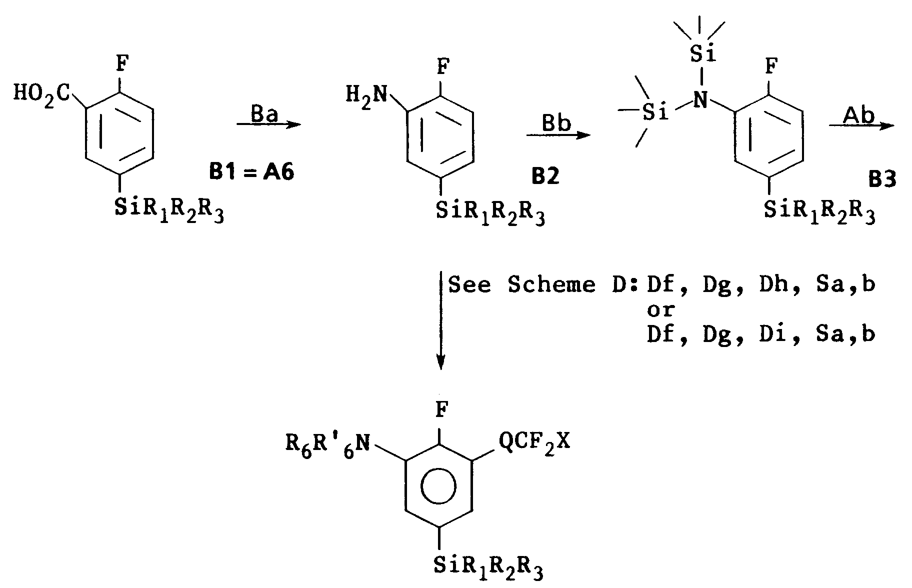

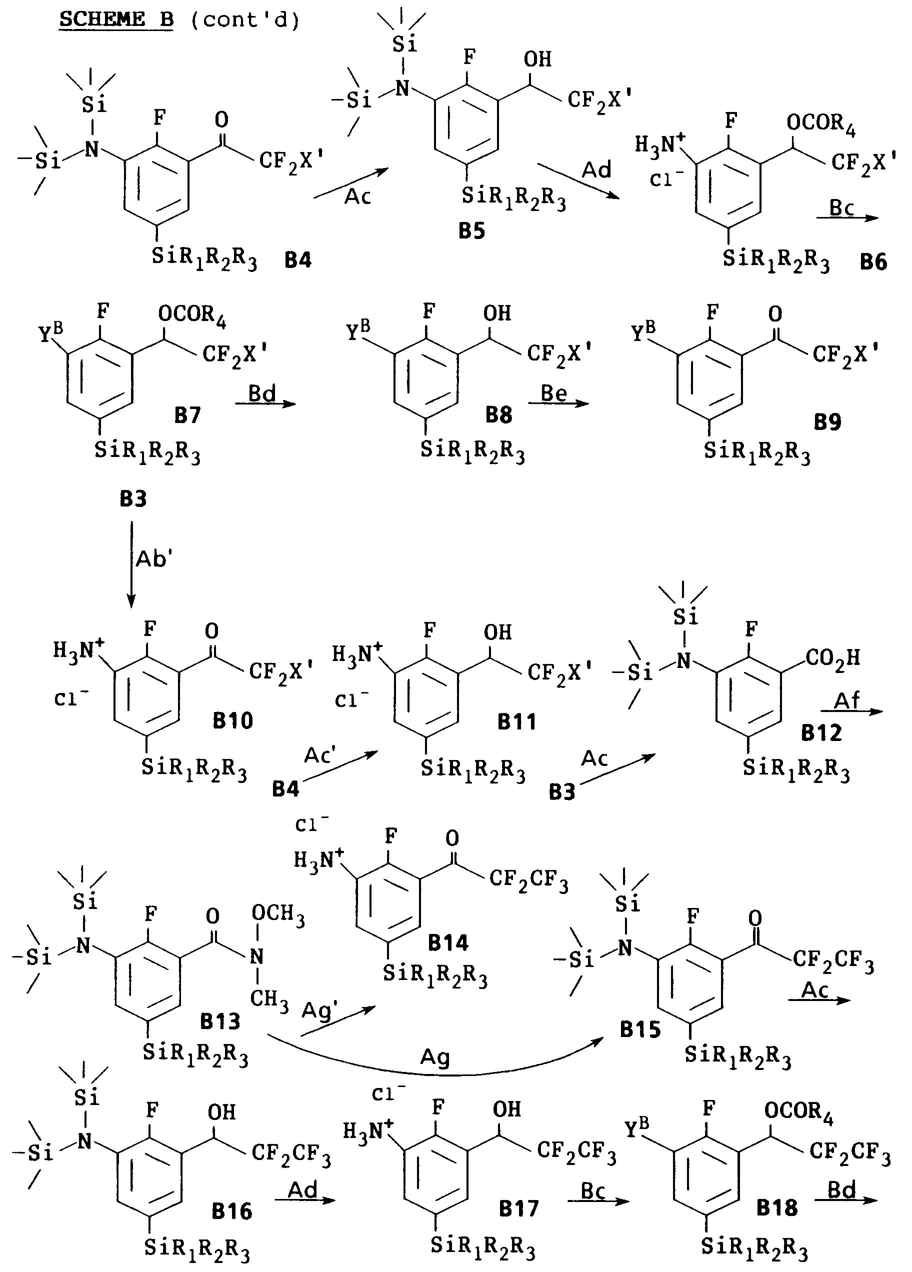

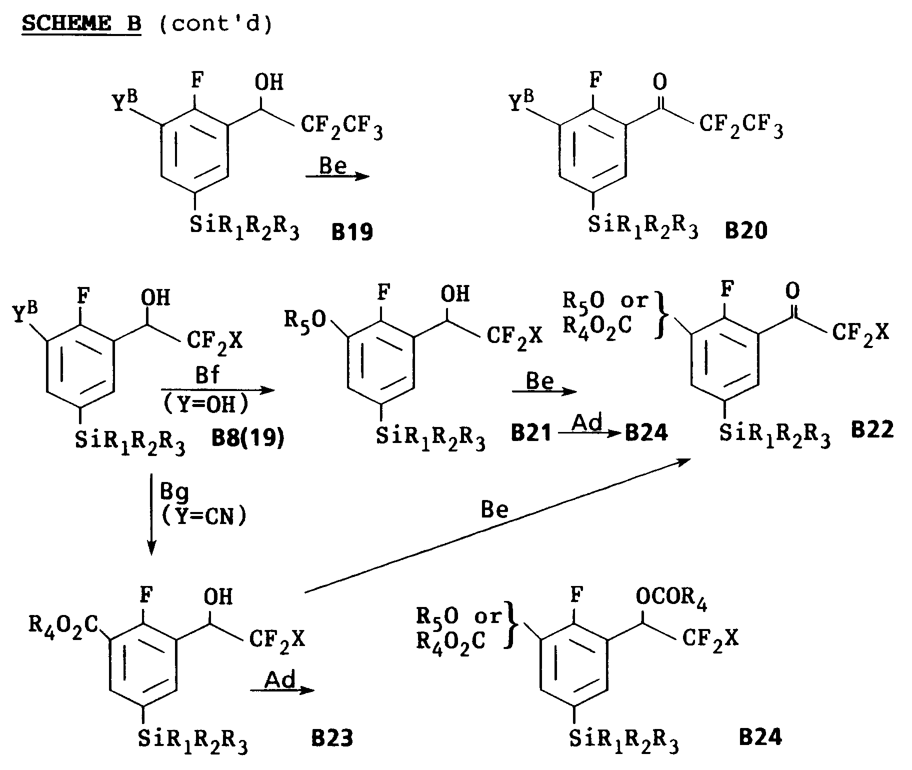

### Reactions:

### Ba: B₁(A₆)→B₂

Carboxylic acids A₆ are reacted with excess of thionyl chloride at reflux temperature to produce acyl chlorides which are reacted with sodium aside at 0°C in acetone-water to produce acyl azides which are heated in benzene or toluene at reflux temperature and then treated with hydrochloric acid at reflux temperature to produce amine hydrochloride salts B₂.

### Bb: B₂→B₃

Amine hydrochloride salts B₂ are converted to their free amines with aqueous sodium hydroxide and then treated with two equivalents of alkyllithium followed by two equivalents of chlorotrimethylsilane in diethyl ether or tetrahydrofuran at -60°C to produce bistrimethylsilylated amines B₃.

### Conversion of B₃ to B₄, then to B₅ by Steps Ab and Ac:

### Ad': B₅→B₆

Following the procedure described in Step Ad the resulting esters are treated with a normal solution of hydrochloric acid.

### Bc: B₆→B₇ and B₁₇→B₁₈

When Y^{B} = Cl, CN, or N₃: The amine hydrochloride salts B₆ are treated with sodium nitrite in water to produce diazonium salts which are heated with cuprous chloride to produce chloroderivatives, or treated with cuprous cyanide to produce nitrile derivatives, or treated with sodium azide to produce azides.

When Y^{B} = Br: The amine hydrochloride salts B₆ are converted to their free amines with aqueous sodium bicarbonate. The free amines are dissolved in aqueous hydrobromic acid, treated with sodium nitrite and then with copper powder to produce bromide derivatives.

When Y^{B} = F or NO₂: The amine hydrochloride salts B₆ are converted to their free amines with aqueous sodium bicarbonate. The free amines dissolved in aqueous fluoroboric acid are treated with sodium nitrite. The diazonium fluoroborate salts are filtered and dried. They are then heated to produce fluoro derivatives or treated with sodium nitrite in the presence of copper powder to produce nitro derivatives.

When Y^{B} = OH: After conversion of the amine hydrochloride salts B₆ to their free amines, the amines are dissolved in aqueous sulfuric acid, diazoted with sodium nitrite and heated to produce phenol derivatives.

When Y^{B} = SO₃H: After conversion of the amine hydrochloride salts to their free amines, the amines are dissolved in an aqueous mixture of sulfuric acid and phosphoric acid, diazoted with sodium nitrite and treated with sulfur dioxide. The reaction mixture is poured onto hydrated ferrous sulfate and copper powder to produce sulfinic acid.

### Bd: B₇→B₈ and B₁₈→B₁₉

Esters B₇ are hydrolized with lithium hydroxyde in aqueous dimethoxyethane to produce alcohols B₈.

### Be: B₈→B₉ and B₁₉→B₂₀

Alcohols B₈ are oxidized with pyridinium dichromate or with Dess-Martin periodinane oxydant in dichloromethane or with the Swern Reaction to produce ketones B₉.

### Ab': B₃→B₁₀ and Ac': B₄→B₁₁ and Ag': B₁₃→B₁₄

Procedure Ab, Ac or Ag, except hydrolysis which is performed with a normal solution of hydrochloric acid.

### Bf: B₈ or B₁₉→B₂₁

Phenol derivatives B₈ or B₁₉ are converted to their sodium or potassium salt with sodium or potassium hydroxyde, or sodium or potassium carbonate in water or acetone and reacted with alkyl bromide or iodide to produce C₁₋₆ alkoxy derivatives.

### Bg: B₈ or B₁₉→B₂₃

Nitrile derivatives are heated in aqueous hydrochloric acid to produce acids (R₄ = H) or treated with an alcohol saturated with dry hydrochloric acid followed by hydrolysis with aqueous sodium bicarbonate to produce esters' (R₄ different from H).

### SCHEME C: to make the subgeneric Formula IV:

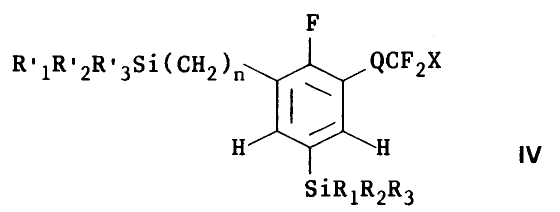

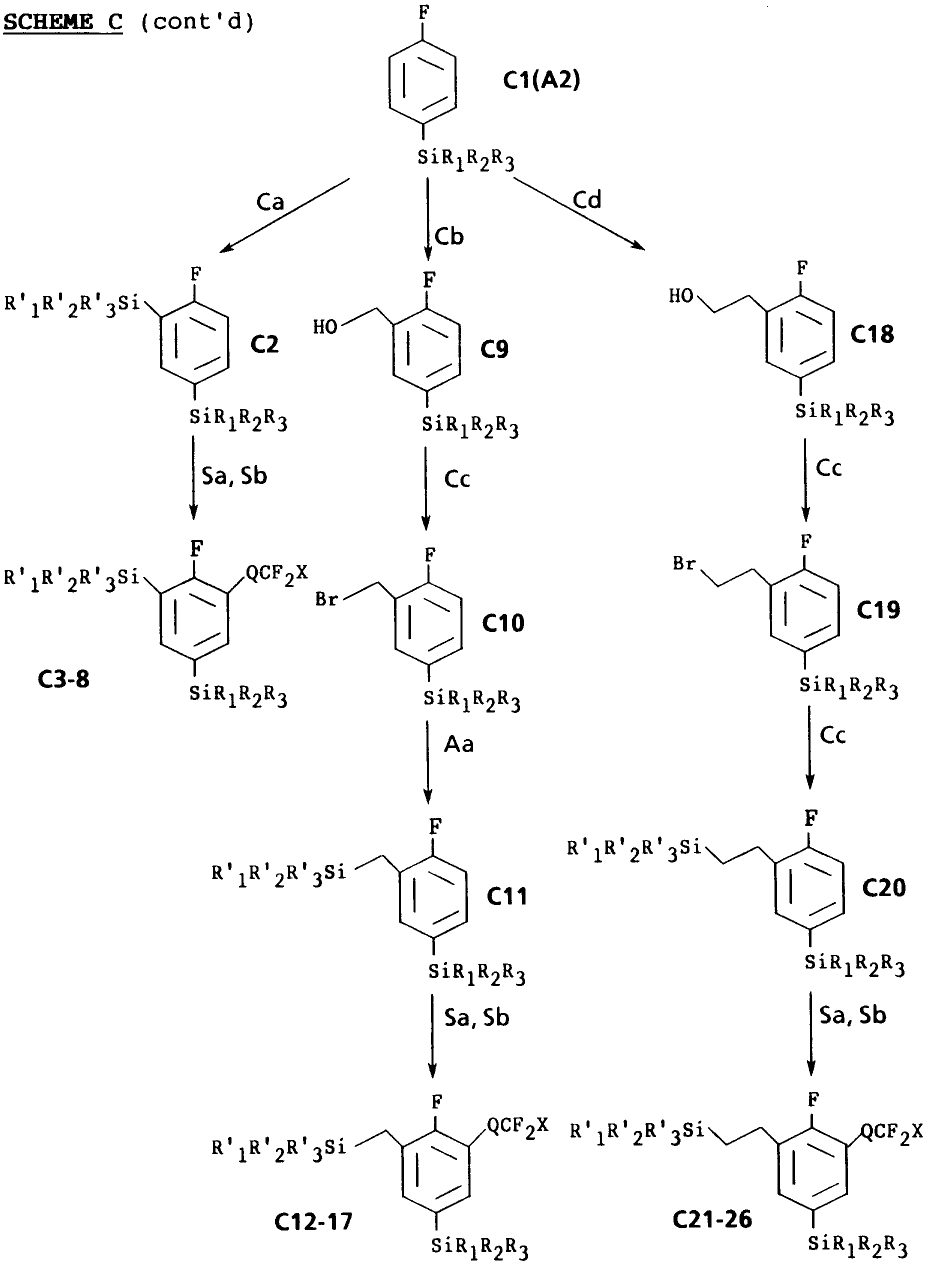

### Ca: C₁(A₂)→C₂:

Following the procedure described in Step Ab the lithium salts are treated with chlorotrisubstituted silane to produce bis silylated derivatives C₂.

### Cb: C₁→C₉:

Following the procedure described in Step Ab the lithium salts are treated with paraformaldehyde to produce benzyl alcohol derivatives C₉.

### Cc: C₉→C₁₀:

Benzyl alcohols are heated with phosphorous tribromide to produce benzyl bromide derivatives C₁₀.

### Cd: C₁→C₁₈:

Following the procedure described in Step Ab the lithium salts are treated with ethylene oxide to produce phenethyl alcohol derivatives C₁₈.

### SCHEME D: to make the subgeneric Formula V:

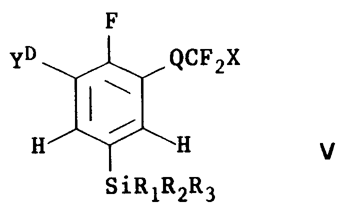

Y^{D} = (C₁₋₆) alkyl, COR₆, hydroxy (C₁₋₆) alkyl, (CH₂)ₙNR₆R₆', or (CH₂)ₘOR₅.
Ts = Tosyl and derivatives thereof. 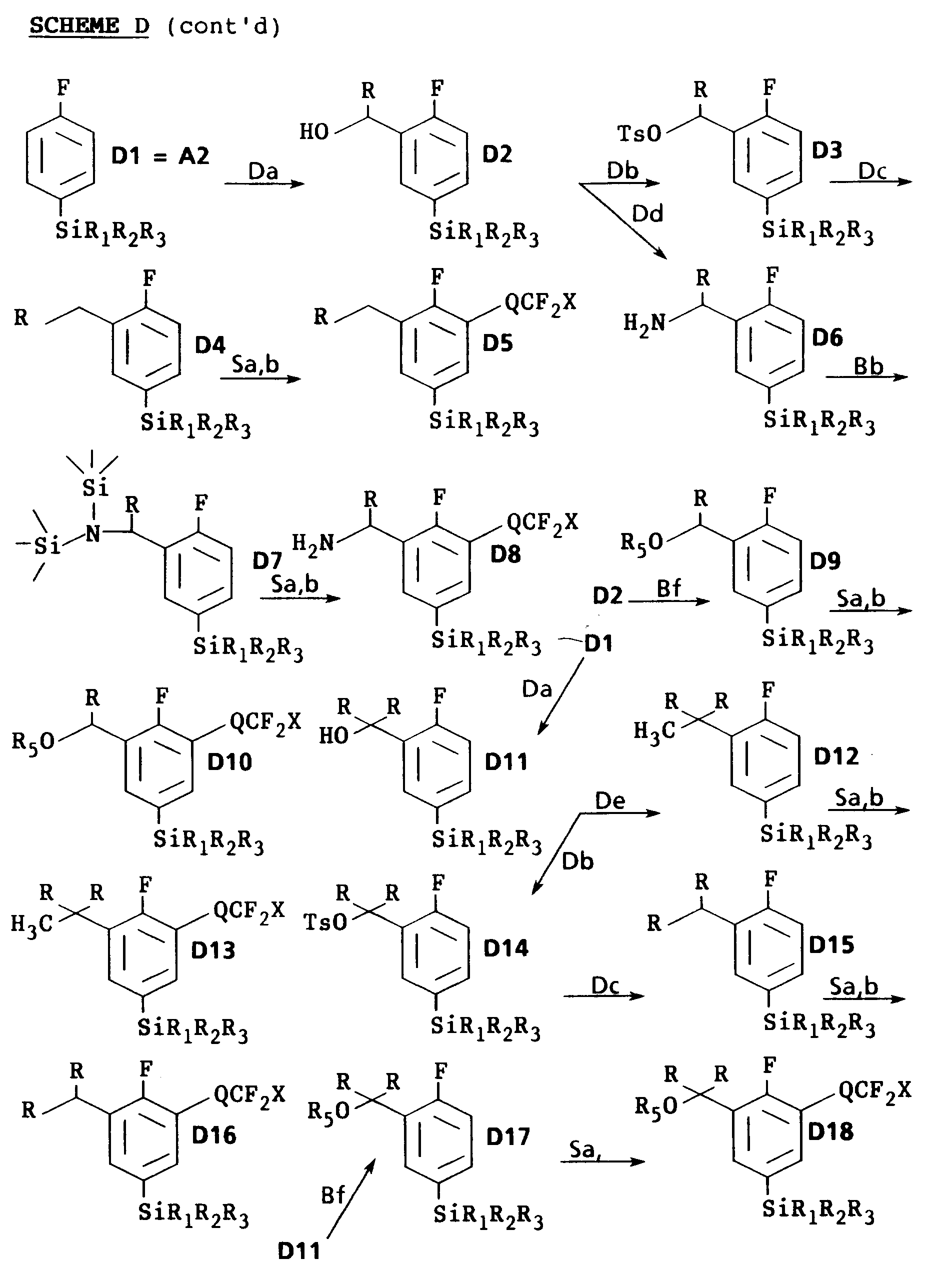 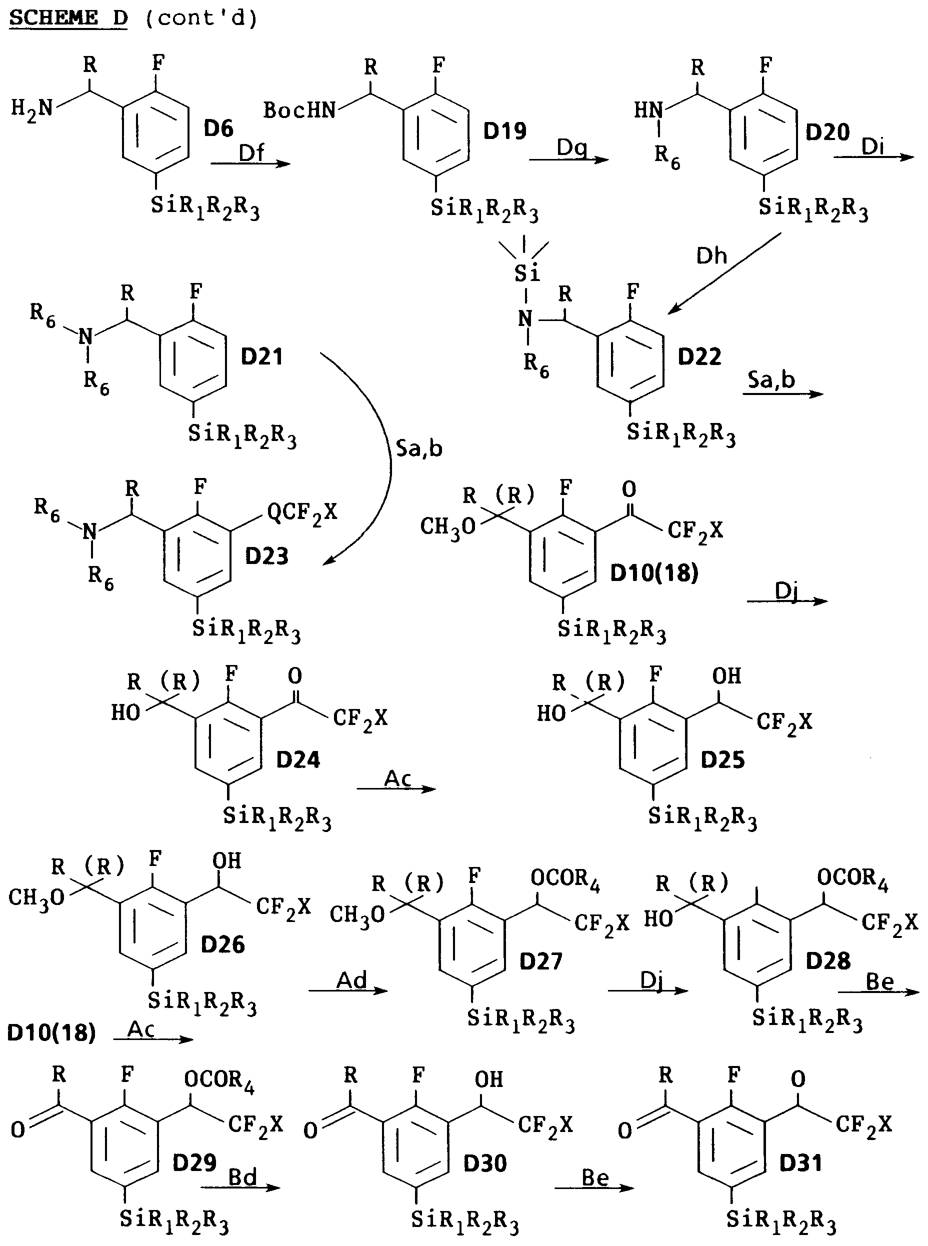
R is H or (C₁₋₁₀) alkyl

### Da: D₁=A₂→D₂or D₁₁:

Following the procedure described in Step Ab the lithium salts are treated with paraformaldehyde or aldehydes or ketones to produce benzyl alcohol derivatives D₂ or D₁₁.

### Db: D₂→D₃ and D₁₁→D₁₄

Benzyl alcohol derivatives D₂ or D₁₁ are treated with p-toluenesulfonylchloride in pyridine at 0°C to produce tosyl derivatives D₃ or D₁₄.

### Dc: D₃→D₄ and D₁₄→D₁₅

Tosyl derivatives are reduced with lithium aluminum hydride in di-n-butyl ether to produce alkyl derivatives D₄ or D₁₅.

### Dc':

Alternatively non-branched alkyl derivatives D₄ are produced by reaction of the lithium salt of A₂ (prepared as described in Step Ab) with a non-substituted alkyl bromide or iodide.

### Dd: D₂→D₆

Benzyl alcohol derivatives D₂ are reacted with phthalimide in the presence of equimolar amounts of diethyl azodicarboxylate and triphenylphosphine in tetrahydrofuran to produce phtalimide derivatives which are then treated with hydrazine hydrate in ethanol or methanol to produce amine derivatives D₆.

### De: D₁₁→D₁₂

Benzyl alcohol derivatives D₁ are heated with trimethyl aluminum to produce alkyl derivatives D₁₂.

### Df: D₆→D₁₉

Amine derivatives D₆ are reacted with di-tert-butyl dicarbonate with one equivalent of triethylamine in dichloromethane to produce N-Boc derivatives D₁₉.

### Dg: D₁₉→D₂₀

N-Boc derivatives D₁₉ are reacted with one equivalent of sodium hydride in tetrahydrofuran. The sodium salt intermediates are reacted with a bromo or iodo derivative of R₆ followed by hydrolysis with aqueous hydrochloric acid. Amine derivatives D₂₀ are purified as their free bases after neutralization of the aqueous phase.

### Dh: D₂₀→D₂₂

Following Step Bb amine derivatives D₂₀ are treated with one equivalent of alkyl lithium reagent followed by one equivalent of chlorotrimethylsilane to produce N-trimethylsilylamine derivatives D₂₂.

### Di: D₂₀→D₂₁

Following Step Dg amine derivatives D₂₀ are treated with one equivalent of sodium hydride and one equivalent of bromo or iodo derivative of R₆ followed by hydrolysis with water to produce amine derivatives D₂₁.

### Dj: D₁₀(D₁₈)→D₂₄ and D₂₇→D₂₈

Methylbenzyl ether derivatives D₉(D₁₇) or D₂₇ are treated with boron tribromide or trimethylsilyliodide in dichloromethane to produce benzyl alcohols derivatives D₂₄ or D₂₈.

### SCHEME E1: to make the subgeneric Formula VI:

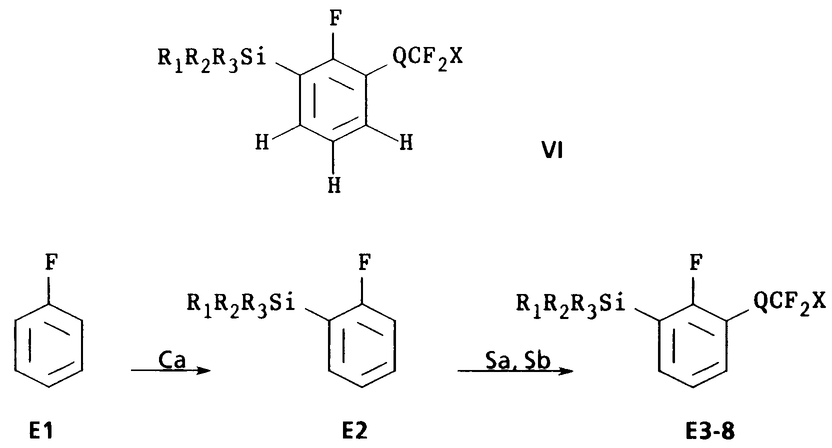

**Ca:** The same reaction is used as previously described in Scheme C, but with the starting materials shown in this scheme.
**Sa, Sb:** Using the starting material shown in Scheme E1, the reaction is as described in Scheme A.

### SCHEME E2: to make the subgeneric Formula VII:

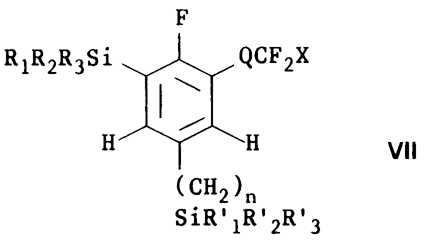

- n =: 1 or 2

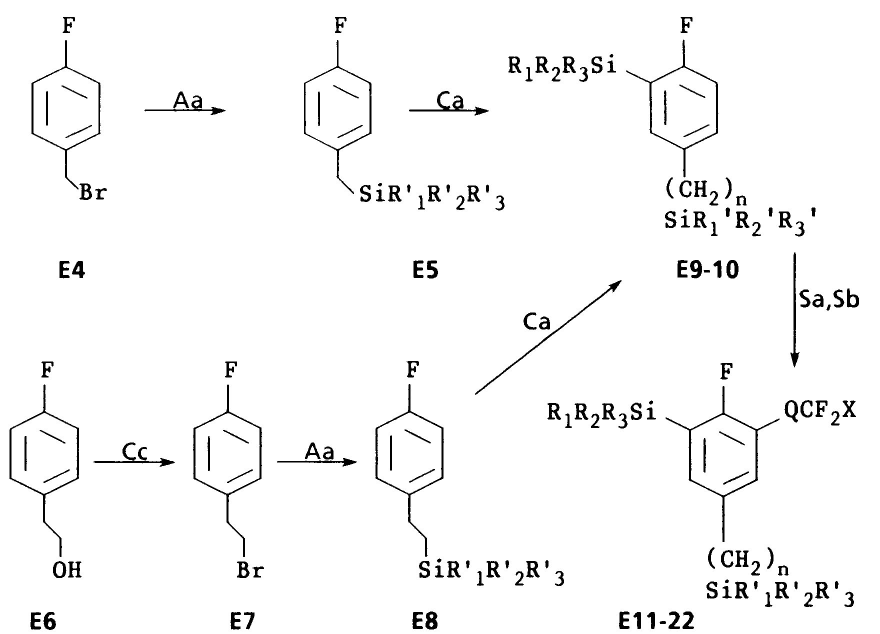

All reactions are as previously defined herein using the compounds shown above.

### SCHEME E3: to make the subgeneric Formula VIII:

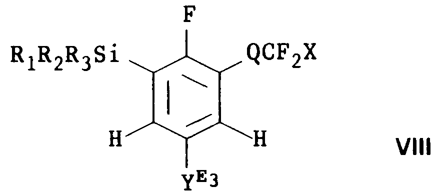

Y 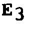 = C₁₋₆ Alkyl, COR₄,
Hydroxyalkyl,
Aminoalkyl,
Alkoxyalkyl,
N-Alkyl, or
N,N-Dialkylaminoalkyl
Ts = tosyl or tosyl derivatives,
R = C₁₋₆ Alkyl. 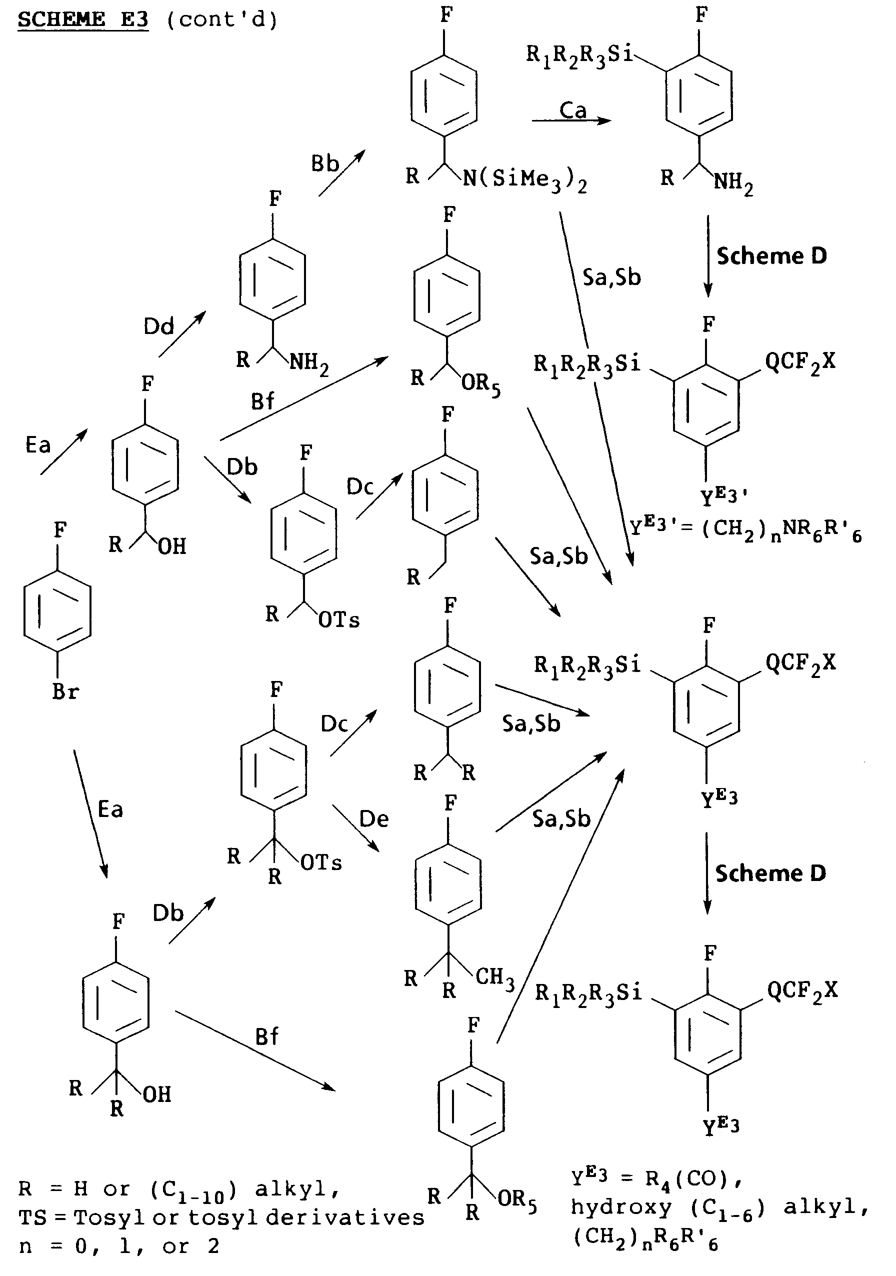
**Ea:** 4-Fluoro-1-bromobenzene is reacted with one equivalent of magnesium in diethyl ether or tetrahydrofuran. To this Grignard intermediate is added paraformaldehyde or aldehyde or ketone to produce benzyl alcohol derivatives.

### SCHEME H: to make the subgeneric Formula IX:

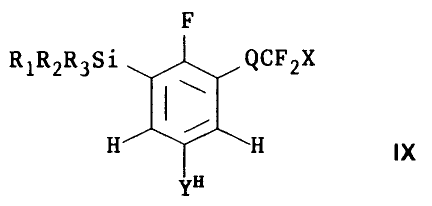

wherein
Y^{H} = OH, NH₂, NR₆R'₆, NO₂, N₃, CN, SO₃H, CO₂R₄, Br, Cl, F, or OR₅;

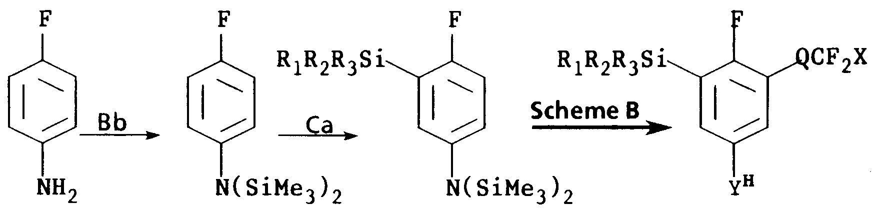

### SCHEME J: to make the subgeneric Formula X:

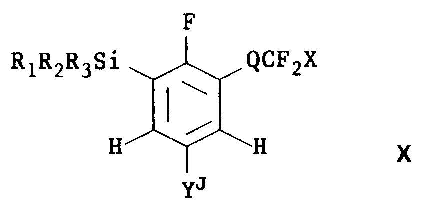

wherein
- Y^{J} =: H or F.

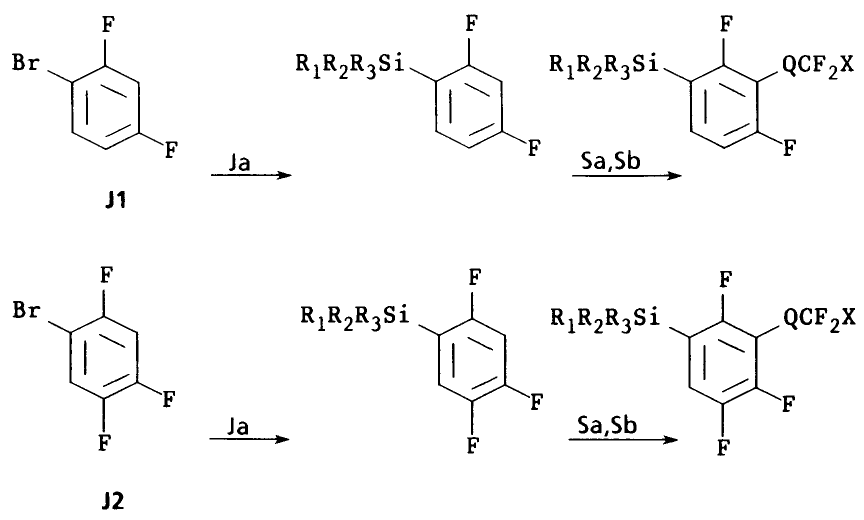

**Ja:** 2,4-Difluoro-1-bromobenzene or 2,4,5-trifluoro-1-bromobenzene is treated with one equivalent of an alkyllithium reagent in diethyl ether or tetrahydrofuran at -78°C. The lithium salt intermediate is reacted with a chlorotri-substituted silane at -50°C to produce the corresponding silylated derivatives.
**Sa, Sb:** As previously described using the compounds shown in Scheme J.

### EXAMPLE 1

### 2,2,2-Trifluoro-1-(3-Trimethylsilyl-6-fluoro)phenyl ethanone

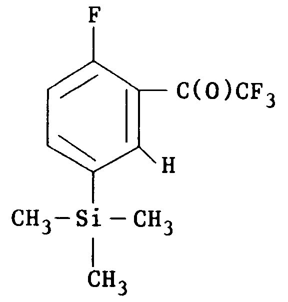

### STEP A:

### 4-TRIMETHYLSILYL-1-FLUOROBENZENE

A solution of 17.5 g (100 mmol) of 4-bromo-1-fluorobenzene and 10.86 g (100 mmol) of chlorotrimethylsilane in 100 ml of tetrahydrofuran was added dropwise in 1.5 hour on 2.43 g (100 mg-atoms) of magnesium in 50 ml of tetrahydrofuran at reflux. Then the reaction mixture was refluxed 18 hours, cooled to 0°C and hydrolized by the addition of 100 ml of 3N HCl. The organic layer was separated, washed with brine, dried over MgSO₄ and concentrated. 4-trimethyl-1-fluorobenzene was distilled to yield 13.45 g (80%); b.p. 64-65°C/15 mmHg.

### STEP B:

### 2,2,2-TRIFLUORO-1-(3-TRIMETHYLSILYL-6-FLUORO)PHENYL ETHANONE

To a solution of 8.40 g (50 mmol) of 4-trimethylsilyl-1-fluorobenzene in 50 ml of tetrahydrofuran at -50°C was added dropwise 33.33 ml (50 mmol) of 1.5M n-butyl lithium in hexane. The reaction mixture was stirred 2 hours and then cooled to -78°C. A solution of 14.20 g (100 mmol) of ethyl trifluoroacetate in 40 ml of tetrahydrofuran was added dropwise and the reaction mixture was stirred 1 hour at -78°C. The cooling bath was removed and when the temperature rose to 0°C, 100 ml of 3N HCl was added. The organic layer was separated, washed with brine, dried over MgSO₄ and concentrated. Chromatography on silica gel (2% of ethyl acetate in petroleum ether) followed by distillation afforded 1.87 g (14%) of title compound; b.p. 120°C/ 14 mmHg.

### EXAMPLE 2

### 2,2,2-Trifluoro-1-(3-trimethylsilyl-6-fluoro)phenyl ethanol

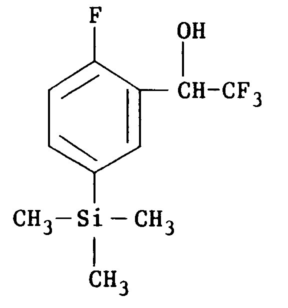

To a solution of 0.67 g (2.55 mmol) of 2,2,2-trifluoro-1-(3-trimethyl-6-fluoro)phenyl ethanone in 5 ml of ethanol at 0°C was added 0.97 g (2.55 mmol) of sodium borohydride. The reaction mixture was stirred 1 hour at room temperature, cooled to 0°C and hydrolized with 1.64 g (30.65 mmol) of ammonium chloride in 30 ml of water. The crude product was extracted with diethyl ether (2 x 30 ml), the organic layer was washed twice with brine, dried over MgSO₄ and concentrated. Chromatography on silica gel (5% of ethyl acetate in petroleum ether) followed by distillation afforded 0.54 g (80%) of the title compound; b.p. 145°C/19 mmHg.

### EXAMPLE 3

### 2,2,2-Trifluoro-1-(2-fluoro-5-dimethylethylsilyl)phenyl ethanone

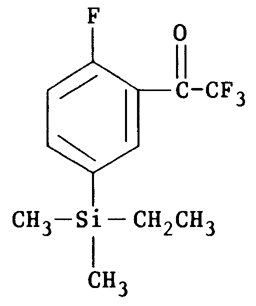

### STEP A:

### 4-DIMETHYLETHYLSILYL-1-FLUOROBENZENE

Following the procedure described in Step A of the Example 1, title compound was obtained in 37% yield; b.p. 85-87°C/17 mmHg.

### STEP B:

### 2,2,2-TRIFLUORO-1-(2-FLUORO-5-DIMETHYLETHYLSILYL)PHENYL ETHANONE

Following the procedure described in Step B of the Example 1, title compound was obtained in 19% yield; b.p. 135°C/14 mmHg.

### EXAMPLE 4

### 2,2,2-Trifluoro-1-(2-fluoro-3-trimethylsilyl)phenyl ethanone

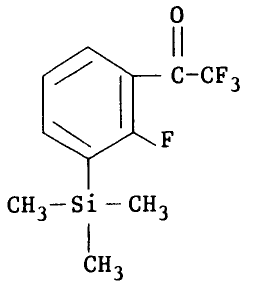

### STEP A

### 2-TRIMETHYLSILYL-1-FLUOROBENZENE

To a solution of 4.8 g (50 mmol) of fluorobenzene in 50 ml of tetrahydrofuran at -50°C was added dropwise 37.60 ml (50 mmol) of 1.33 M n-butyl lithium hexane. The reaction mixture was stirred 6 hours while the temperature was kept between -40°C and -50°C. To the solution was added 5.43 g (50 mmol) of chlorotrimethylsilane in 10 ml of tetrahydrofuran. The reaction mixture was stirred 3 hours at -50°C, 15 hours at room temperature and hydrolized with 5.35 g (100 mmol) of ammonium chloride in 50 ml of water. The organic layer was separated, washed with brine, dried over MgSO₄ and concentrated. Crude material was distilled *in vacuo,* yielding 0.37 g (3%); b.p. 42°C/15 mmHg.

### STEP B:

### 2,2,2-TRIFLUORO-1-(2-FLUORO-3-TRIMETHYLSILYL)PHENYL ETHANONE

To a solution of 0.37 g (1.6 mmol) of 2-trimethylsilyl-1-fluorobenzene in 3.5 ml (1.6 mmol) of 1.6 M n-butyl lithium in hexane. The reaction mixture was stirred 6 hours at -50°C, cooled to -78°C and a solution of 0.23 g (1.6 mmol) of ethyl trifluoroacetate in 2 ml of tetrahydrofuran was added. The reaction mixture was stirred one hour at -78°C, 15 hours at room temperature and hydrolized with 5 ml of 1 N HCl. The organic layer was separated, washed with brine, dried over MgSO₄ and concentrated. Chromatograph on silica gel (5% of ethyl acetate in petroleum ether) afforded 0.12 g (28%) of the title compound.

### EXAMPLE 5

### 2,2,2-Trifluoro-1-(2,6-difluoro-3-trimethylsilyl)phenyl ethanone

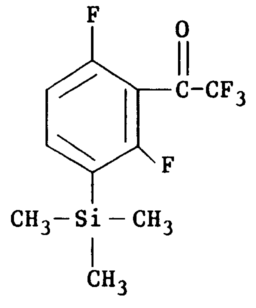

### STEP A:

### 2,4-DIFLUORO-1-TRIMETHYLSILYLBENZENE

To a solution of 5.8 g (30 mmol) of 2,4-difluoro-1-bromobenzene in 40 ml of tetrahydrofuran at -78°C was added dropwise 20 ml (30 mmol) of 1.5 M n-butyl lithium in hexane. The reaction mixture was stirred 10 minutes at -78°C. Then a solution of 3.26 g (30 mmol) of chlorotrimethylsilane in 15 ml of tetrahydrofuran was added dropwise and the reaction mixture was stirred 7 hours between -60°C and -50°C. To the reaction mixture was added 4.8 g (90 mmol) of ammonium chloride in 30 ml of water and the organic layer was separated, washed with brine, dried over MgSO₄ and concentrated. Title compound was purified by distillation.

### STEP B:

### 2,2,2-TRIFLUORO-1-(2,6-DIFLUORO-3-TRIMETHYLSILYL)PHENYL ETHANONE

Title compound was prepared as described in step B of the Example 4 and purified by distillation.

### EXAMPLE 6

### 2,2,2-Trifluoro-1-(2-fluoro-3-methoxymethyl-5-trimethylsilyl)phenyl ethanone

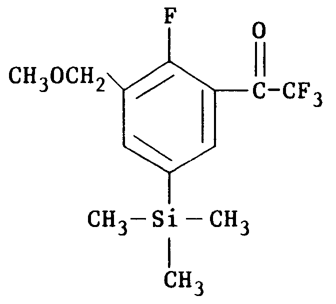

### STEP A:

### 2-HYDROXYMETHYL-5-TRIMETHYLSILYL-1-FLUOROBENZENE

To a solution of 3.36 g (20 mmol) of 4-fluoro-1-trimethylsilyl benzene in 20 ml of tetrahydrofuran at -50°C was added dropwise 13.33 ml (20 mmol) of 1.5 M n-butyl lithium in hexane. The reaction mixture was stirred 6 hours while the temperature was kept between -50°C and -60°C. To the solution was added 1.20 g (40 mmol) of paraformaldehyde by portion at -78°C. The reaction mixture was stirred one hour at -78°C, 15 hours at room temperature and hydrolized with 20 ml of 3N HCl. The organic layer was separated, washed with brine, dried over MgSO₄ and concentrated. The title compound was purified by distillation.

### STEP B:

### 2-METOXYMETHYL-5-TRIMETHYLSILYL-1-FLUOROBENZENE

A solution of 1.98 g (10 mmol) of 2-hydroxymethyl-5-trimethylsilyl-1-fluorobenzene in 10 ml of tetrahydrofuran was added dropwise on a mixture of 0.24 g (10 mmol) of sodium hydride in 10 ml of tetrahydrofuran at 0°C. The reaction mixture was stirred 3 hours at room temperature and cooled to 0°C. A solution of 1.42 g (10 mmol) of iodomethane in 10 ml of tetrahydrofuran was added dropwise and the reaction mixture was stirred 15 hours at room temperature. A solution of 1.60 g (30 mmol) of ammonium chloride in 10 ml of water was added, the organic layer was separated, washed with brine, dried over MgSO₄ and concentrated. The title compound was purified by distillation.

### STEP C:

### 2,2,2-TRIFLUORO-1-(2-FLUORO-3-METHOXYMETHYL-5-TRIMETHYLSILYL)PHENYL ETHANONE

Title compound was prepared as described in step B of Example 4.

### EXAMPLE 7

### 2,2,2-Trifluoro-1-(2-fluoro-3-hydroxymethyl-5-trimethylsilyl)phenyl ethanone

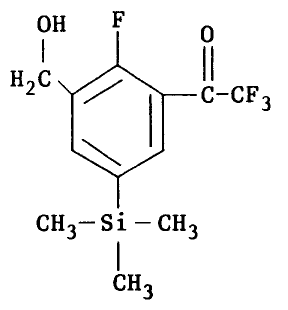

To a solution of 1.54 g (5 mmol) of 2,2,2-trifluoro-1-(2-fluoro-3-methoxymethyl-5-trimethylsilyl)phenyl ethanone in 10 ml of dichloromethane at -78°C was added dropwise 5 ml (5 mmol) if 1M boron tribromide in dichloromethane. Cooling bath was removed and the reaction mixture was stirred one hour at room temperature. Then 2 ml of methanol was added dropwise, followed by 10 ml of water. The organic layer was separated, washed with brine, dried over MgSO₄ and concentrated. Title compound was recrystallised from isopropanol.

### EXAMPLE 8

### 2,2,2-Trifluoro-1-(2-fluoro-3-n-butyl-5-trimethylsilyl)phenyl ethanone

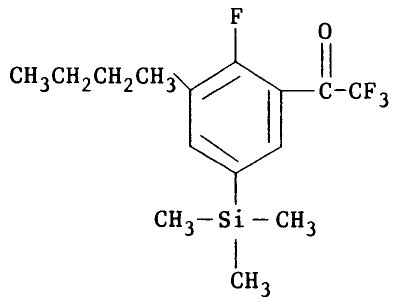

### STEP A:

### 2-n-BUTYL-4-TRIMETHYLSILYL-1-FLUOROBENZENE

To a solution of 3.36 g (20 mmol) of 4-fluoro-1-trimethylsilylbenzene in 20 ml of tetrahydrofuran at -50°C was added dropwise 13.33 ml (20 mmol) of 1.5 M n-butyl lithium in hexane. The reaction mixture was stirred 4 hours between -40°C and -50°C and then 3.68 g (20 mmol) of n-iodobutane in 10 ml of tetrahydrofuran was added dropwise. The reaction mixture was stirred 4 hours between -40°C and -50°C, cooling bath was removed and 20 ml of 1N HCl was added dropwise. The organic layer was separated, washed with brine, dried over MgSO₄ and concentrated. The title compound was purified by distillation.

### STEP B:

### 2,2,2-TRIFLUORO-1-(2-FLUORO-3-n-BUTYL-5-TRIMETHYLSILYL)PHENYL ETHANONE

Title compound was prepared as described in step B of the Example 4.

### EXAMPLE 9

### 2,2,2-Trifluoro-1-(2-fluoro-3-amino-5-trimethylsilyl)phenyl ethanone hydrochloride

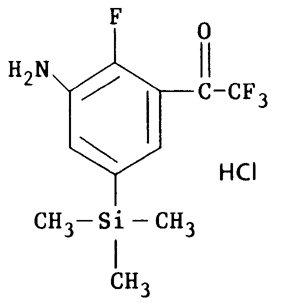

### STEP A:

### 2-FLUORO-5-TRIMETHYLSILYL-BENZOIC ACID

To a solution of 8.4 g (50 mmol) of 4-fluoro-1-trimethylsilyl benzene in 50 ml of tetrahydrofuran at -60°C was added dropwise 31.25 ml (50 mmol) of 1.6 M n-butyl lithium in hexane and the reaction mixture was stirred 6 hours between -50°C and -60°C. Then the reaction mixture was treated with excess of carbon dioxide, cooling bath was removed and 50 ml of water was added dropwise. Tetrahydrofuran was removed under reduced pressure and the aqueous solution was extracted twice with 30 ml of n-hexane. The aqueous layer was acidified with 20 ml of 6N HCl, extracted twice with 50 ml of ethyl acetate. The organic layers were combined, washed with brine, dried over MgSO₄ and concentrated. Title compound was recrystallized from isopropanol.

### STEP B:

### 2-FLUORO-5-TRIMETHYLSILYL-ANILINE

A mixture of 2.12 g (10 mmol) of 2-fluoro-5-trimethylsilyl-benzoic acid and 1.78 g (15 mmol) of thionyl chloride was heated 2 hours at 60°C. Then gases and excess of thionyl chloride were removed under reduced pressure. To the crude material dissolved in 10 ml of acetone was added dropwise 6.5 g (10 mmol) of sodium aside in 10 ml of water and the mixture was stirred one hour at 0°C. Acetone was removed under reduced pressure and the acyl azide was extracted with ethyl acetate. The organic phase was dried over MgSO₄ and ethyl acetate was removed under reduced pressure. To the acyl azide 20 ml of benzene was added and the reaction mixture was stirred 30 minutes at reflux. Then the solution was cooled to 0°C, 10 ml of concentrated hydrochloric acid was added and the mixture was heated 30 minutes at reflux. Benzene and water were removed under reduced pressure and the hydrochloride salt was recrystallized from isopropanol. To the salt dissolved in 10 ml of water was added 10 ml of 1N sodium hydroxide and the aqueous mixture was extracted with ethyl acetate. The organic layer was washed with brine, dried over MgSO₄ and concentrated to obtain pure 2-fluoro-5-trimethylsilyl-aniline.

### STEP C:

### 2-FLUORO-5-TRIMETHYLSILYL-N,N-(bis-TRIMETHYLSILYL)ANILINE

To a solution of 0.91 g (5 mmol) of 2-fluoro-5-trimethylsilyl-aniline in 10 ml of tetrahydrofuran at 0°C was added dropwise 6.67 ml (10 mmol) of 1.5 M n-butyl lithium in hexane and 10 minutes later 1.08 g (10 mmol) of chlorotrimethylsilane in 10 ml of tetrahydrofuran. Cooling bath was removed and the mixture was stirred one hour at reflux. Solvents were removed under reduced pressure and title compound was purified by distillation.

### STEP D:

### 2,2,2-TRIFLUORO-1-(2-FLUORO-3-AMINO-5-TRIMETHYLSILYL)PHENYL ETHANONE HYDROCHLORIDE

To a solution of 0.98 g (3 mmol) of 2-fluoro-5-trimethylsilyl-N,N-(bis-trimethylsilyl)aniline in 6 ml of tetrahydrofuran at -60°C was added dropwise 2 ml (3 mmol) of 1.5 M n-butyl lithium in hexane. The reaction mixture was stirred 6 hours between -50°C and -60°C, cooled to -78°C and a solution of 0.85 g (6 mmol) of ethyl trifluoroacetate in 6 ml of tetrahydrofuran was added dropwise. The reaction mixture was stirred one hour at -78°C, 15 hours at room temperature and hydrolized with 10 ml of 3N HCl. Organic solvents were removed under reduced pressure. The aqueous layer was extracted twice with diethyl ether, basified with 10 ml of 6 N sodium hydroxide, extracted twice with ethyl acetate. The ethyl acetate layers were combined, washed with brine, dried over MgSO₄ and concentrated. Crude material was dissolved in diethyl ether and treated with a saturated solution of HCl in diethyl ether. The hydrochloride salt was filtered and recrystallised from isopropanol.

It is now established that Alzheimer's disease and other senile degenerative diseases such as senile dementia are characterised by a selective loss in the cerebral cortex of choline acetyltransferase, the enzyme responsible for the biosynthesis of acetylcholine. There also exists a good correlation between memory impairment or dementia and the decrement in cholinergic transmission. Thus, impaired cholinergic transmission in the central nervous system may be, at least in part, responsible for the symptomatology of Alzheimer's disease and senile dementia. In support to these conclusions such compounds as physostigmine and 1,2,3,4-tetrahydro-9-aminoacridine (THA), compounds which prevent the catabolism of acetylcholine have found a place in the treatment of Alzheimer's and other senile degenerative diseases. Indeed, it has been recognized that the extent of improvement of cognitive functions has been closely related to the degree of inhibition of acetylcholinesterase.

The compounds of the present invention are useful in treating other conditions responsive to inhibition of acetylcholinesterase such as Myasthenia Gravis [*J.Neurol*. *Neurosurg. Psychiatry,* 46 (10) 1983, 929-935, *Neurology* 42 (6) 1992, 1153-1156], antidotes against poisoning with organo-phosphates [see USP No.5,171,750, *Int. J. Clin. Pharmacol. Ther. Toxicol.* 27 (8) 1989, 367-387], and glaucoma (*Arch. Clin. Exp. Ophthalmol.* 229 (3), 1991, 252-253).

The compounds of Formula I are pharmacologically active agents capable of inhibiting acetylcholinesterase as demonstrable in standard biological *in vitro* and *in vivo* test procedures. Indeed, based upon standard laboratory procedures, it is to be shown that the compounds of Formula I are potent and selective, quasi irreversible inhibitors of acetylcholinesterase capable of demonstrating advantages over the prior art, particularly physostigmine, in their use in the treatment of Alzheimer's disease and senile dementia. The compounds, in general, will exert their acetylcholinesterase inhibitory properties within the dose range of about 0.01 mg to 5 mg per kilogram of body weight for the preferred compounds.

For pharmacological end-use applications, the compounds of Formula I are preferentially administered in the form of their pharmaceutically acceptable acid addition salts. Of course, the effective dosage of the compounds will vary according to the individual potency of each compound employed, the severity and nature of the disease being treated and the particular subject being treated. In general, effective results can be achieved by administering a compound at a dosage of about 0.01 mg to about 20 mg per kilogram of body weight per day, administered systemically. Therapy should be initiated at lower dosages. The dosage thereafter may be administered orally in solid dosage forms, e.g., capsules, tablets, or powders, or in liquid forms, e.g., solutions or suspensions. The compounds may also be injected parenterally in the form of sterile solutions or suspensions.

In practicing the method of this invention, the active ingredient is preferably incorporated in a composition comprising a pharmaceutical carrier and from about 5 to about 90 percent by weight of a compound of the invention or a pharmaceutically-acceptable salt thereof. The term "pharmaceutical carrier" refers to known pharmaceutical excipients useful in formulating pharmaceutically active compounds for internal administration to animals, and which are substantially non-toxic and non-sensitising under conditions of use. The compositions can be prepared by known techniques for the preparation of tablets, capsules, elixirs, syrups, emulsions, dispersions and wettable and effervescent powders, and can contain suitable excipients known to be useful in the preparation of the particular type of composition desired.

The preferred route of administration is oral administration. For oral administration the formula I compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptable surfactant, suspending agent, or emulsifying agent.

The formula I compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as polyethylene glycol 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically acceptable adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl beta-aminopropionates, and 2-alkylimidazoline quarternary ammonium salts, as well as mixtures. The parenteral compositions of this invention will typically contain from about 0.5 to about 25% by weight of the formula I compound in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Patent Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Patent No. 3,921,636. At least two types of release are possible in these systems. Release by diffusion occurs when the matrix is non-porous. The pharmaceutically effective compound dissolves in and diffuses through the matrix itself. Release by microporous flow occurs when the pharmaceutically effective compound is transported through a liquid phase in the pores of the matrix.

As is true for most classes of compounds suitable for use as therapeutic agents, certain subgeneric groups and certain specific compounds are preferred. Preferably, R₁, R₂, and R₃ are each methyl or ethyl, or mixtures thereof. Y is preferably hydrogen, -(CH₂)ₙOR₅ wherein R₅ is C₁₋₁₀ alkyl, and more preferably, methyl, hydroxy (C₁₋₆ alkyl and more preferably alpha hydroxy C₁₋₆ alkyl, C₁₋₄ alkyl, or -(CH₂)_{N}NR₆R₆' wherein N preferably is zero. Preferably, Z is F. Q is preferably C(O) or CH(OH). Y is preferably F. Z' is preferably H.

Specifically preferred compounds besides the compounds specifically exemplified are those charted below as follows:

| R₁ | R₂ | R₃ | Z' | Z | X | Y |
|---|---|---|---|---|---|---|
| Methyl | Methyl | Methyl | H | F | F | Methyl |
| Methyl | Methyl | Methyl | F | H | F | Methyl |
| Methyl | Methyl | Methyl | H | F | F | Isopropyl |
| Methyl | Methyl | Methyl | F | H | F | Isopropyl |
| Methyl | Methyl | Ethyl | F | H | F | H |
| Methyl | Methyl | Ethyl | H | F | F | Methyl |
| Methyl | Methyl | Ethyl | F | H | F | Methyl |
| Methyl | Methyl | Ethyl | H | F | F | Isopropyl |
| Methyl | Methyl | Ethyl | F | H | F | Isopropyl |
| Methyl | Ethyl | Ethyl | H | F | F | H |
| Methyl | Ethyl | Ethyl | F | H | F | H |
| Methyl | Ethyl | Ethyl | H | F | F | Methyl |
| Methyl | Ethyl | Ethyl | F | H | F | Methyl |
| Methyl | Ethyl | Ethyl | H | F | F | Isopropyl |
| Methyl | Ethyl | Ethyl | F | H | F | Isopropyl |
| Methyl | Methyl | Propyl | H | F | F | H |
| Methyl | Methyl | Propyl | F | H | F | H |
| Methyl | Methyl | Propyl | H | F | F | Methyl |
| Methyl | Methyl | Propyl | F | H | F | Methyl |
| Methyl | Methyl | Propyl | H | F | F | Isopropyl |
| Methyl | Methyl | Propyl | F | H | F | Isopropyl |

## Claims

1. A compound of the formula: 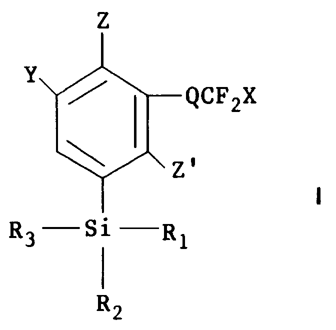 stereoisomers or mixtures thereof, and pharmaceutically acceptable salts thereof, wherein:
each of Z and Z' are independently H or F, provided that at least one of Z or Z' is F;
Q is 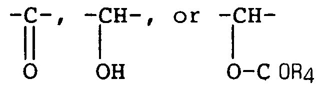 X is H, Br, Cl, F or CF₃;
Y is H, OH, (C₁₋₆) alkyl, -(CH₂)ₘOR₅, hydroxy(C₁₋₆)alkyl, -(CH₂)ₙNR₆R₆', azido, CN, CO₂R₄, COR₆, SO₃H, Br, Cl, F, NO₂ or -(CH₂)ₙSIR₁'R₂'R₃', provided that when both Z and Z' are F, then Y is H or F;
R₁, R₂, R₃, R₁', R₂' and R₃' are each independently C₁₋₁₀ alkyl or (CH₂)ₙaryl;
R₄ is H, C₁₋₁₀ alkyl, phenyl, benzyl or phenethyl;
R₅ is H, C₁₋₁₀ alkyl, benzyl or phenethyl;
R₆ and R₆' are hydrogen or C₁₋₁₀ alkyl;
m is an integer of 0, 1, 2, 3 or 4; and
n is an integer of 0, 1 or 2.

2. The compound of Claim 1 wherein each of R₁, R₂, R₃, R'₁, R'₂ and R'₃, when present are independently methyl.

3. The compound of Claim 1 wherein Z is F.

4. The compound of Claim 1 wherein Z' is H.

5. The compound of Claim 1 wherein Q is C(O) or CH(OH).

6. The compound of Claim 1 wherein the compound is
2,2,2-Trifluoro-1-(3-Trimethylsilyl-6-fluoro)phenyl ethanone;
2,2,2-Trifluoro-1-(3-trimethylsilyl-6-fluoro)phenyl ethanol;
2,2,2-Trifluoro-1-(2-fluoro-5-dimethylethylsilyl)phenyl ethanone;
2,2,2-Trifluoro-1-(2-fluoro-3-trimethylsilyl)phenyl ethanone;
2,2,2-Trifluoro-1-(2,6-difluoro-3-trimethylsilyl)phenyl ethanone;
2,2,2-trifluoro-1-(2-fluoro-3-methoxymethyl-5-trimethylsilyl)phenyl ethanone;
,2,2-trifluoro-1-(2-fluoro-3-hydroxymethyl-5-trimethylsilyl)phenyl ethanone;
2,2,2-trifluoro-1-(2-fluoro-3-n-butyl-5-trimethylsilyl)phenyl ethanone; or
2,2,2-trifluoro-1-(2-fluoro-3-amino-5-trimethylsilyl)phenyl ethanone hydrochloride.

7. A compound according to Claim 1 for use as a pharmaceutically active compound.

8. A compound according to Claim 1 for use in the treatment of Degenerative Dementias.

9. Use of a compound according to Claim 1, optionally in combination with a pharmaceutically acceptable carrier, for the preparation of a pharmaceutical composition for the treatment of Degenerative Dementias.
